# EUROPEAN PATENT APPLICATION

(11) **EP 1 666 471 A1**
(43) Date of publication of application: **07.06.2006**
(21) Application number: 04772886.0
(22) Date of filing: 01.09.2004
(51) Int. Cl.: C07D 257/04

(54) **PROCESS FOR PRODUCING 2' -(1H-TETRAZOL-5-YL)-BIPHENYL-4-CARBALDEHYDE**

(30) Priority: 04.09.2003 JP 2003313325
(71) Applicant: Sumitomo Chemical Company, Limited, Tokyo 104-8260 (JP)
(72) Inventor: ITAYA, Nobushige, Nishinomiya-shi, Hyogo 6620841 (JP); MATSUI, Kozo, Kobe-shi, Hyogo 6500046 (JP); OHTANI, Yutaka, Kobe-shi, Hyogo 6570036 (JP); UENO, Hiroki, Sakai-shi, Osaka 5900024 (JP); KANEKO, Toshikazu, Takatsuki-shi, Osaka 5691126 (JP)
(74) Representative: Vossius & Partner
(86) International application number: PCT/JP2004/013014
(87) International publication number: WO 2005/023785

(57) **Abstract**

The present invention provides a process for producing 2'-(1H-tetrazol-5-yl)biphenyl-4-carbaldehyde which comprises reacting 2'-cyanobiphenyl-4-carbaldehyde with a salt of azide; a process for producing a highly pure crystal of 2'-(1H-tetrazol-5-yl)biphenyl-4-carbaldehyde which comprises reacting 2'-cyanobiphenyl-4-carbaldehyde with a salt of azide, obtaining a crystal of 2'-(1H-tetrazol-5-yl)biphenyl-4-carbaldehyde, dissolving said crystal obtained and recrystallizing a highly pure crystal in tetrahydrofuran; and the like.

According to the present process, 2'-(1H-tetrazol-5-yl)biphenyl-4-carbaldehyde which is useful as a synthetic intermediate of medicines can be produced in a short process at high yield and high purity.

## Description

### TECHNOLOGICAL FIELD

The present invention relates to a process for producing 2'-(1H-tetrazol-5-yl)biphenyl-4-carbaldehyde which is a useful synthetic intermediate for mecidines, particularly, non-peptide angiotensin II antagonist, further, a process for producing a crystal of said compound, and said compound of high purity.

### BACKGROUND TECHNOLOGY

2'-(1H-tetrazol-5-yl)biphenyl-4-carbaldehyde is useful as a synthetic intermediate for angiotensin II antagonist which is an depressor (see, JP-H05-271205-A).

In medicines, impurities should be controlled for ensuring safety, and also 2'-(1H-tetrazol-5-yl)biphenyl-4-carbaldehyde as a synthetic intermediate is required to have high purity.

Particularly, there is such a problem that purity of 2'-(1H-tetrazol-5-yl)biphenyl-4-carbaldehyde lowers as the compound is easily oxidized in solution condition, and contains, as an impurity, 2'-(1H-tetrazol-5-yl)biphenyl-4-carboxylic acid produced.

As a method of producing 2'-(1H-tetrazol-5-yl)biphenyl-4-carbaldehyde, a method shown in the following scheme is known (e.g., JP-H05-271205-A).

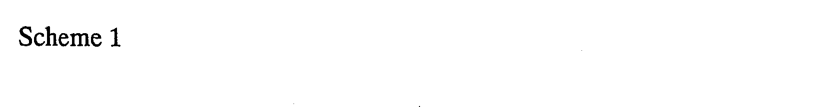

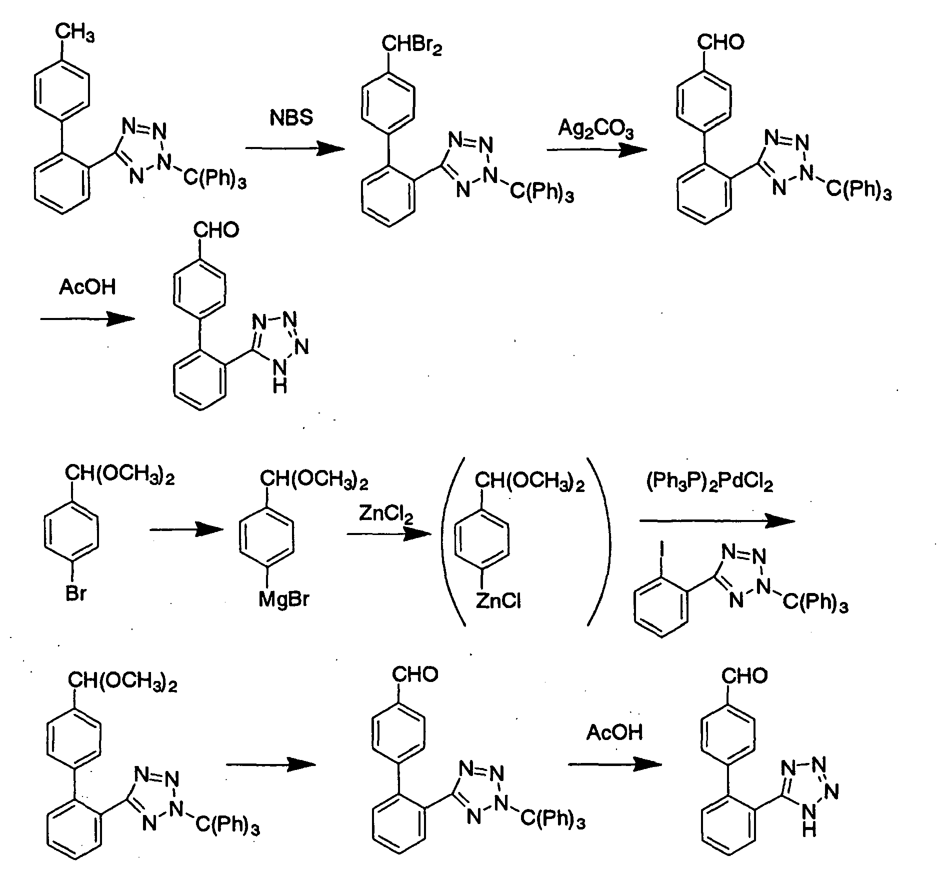

However, N-bromosuccinic imide (NBS) used as a brominating agent, a raw material 4-bromobenzaldehyde dimethylacetal, dichlorobis(triphenylphosphine)palladium used as a catalyst, and the like are expensive and, in addition, the yield is insufficient. Therefore, the above-mentioned method is not satisfactory as an industrial production method.

Further, the resultant 2'-(1H-tetrazol-5-yl)biphenyl-4-carbaldehyde contains impurities such as its oxide, 2'-(1H-tetrazol-5-yl)biphenyl-4-carboxylic acid, and the like, and it has been difficult to obtain 2'-(1H-tetrazol-5-yl)biphenyl-4-carbaldehyde of high purity which is satisfactory as a synthetic intermediate of medicines.

The present invention has been accomplished for solving the above-mentioned problem.

### SUMMARY OF THE INVENTION

The present inventors have intensively studies to solve the above-mentioned problem and resultantly found that 2'-(1H-tetrazol-5-yl)biphenyl-4-carbaldehyde is obtained in high yield by reacting 2'-cyanobiphenyl-4-carboalhyde with an salt of azide such as cheap azide of organic base, azide of inorganic base and the like. Further, the present inventors have found a method of efficiently obtaining a high purity crystal of 2'-(1H-tetrazol-5-yl)biphenyl-4-carbaldehyde and concurrently, found that a crystal obtained by this method shows a novel physical property, leading to completion of the present invention.

That is, the present invention is as described below. (Please check Claims as the followings are almost the same as the Claims backward. It is not necessary to check light blue part.)
<1> A process for producing 2'-(1H-tetrazol-5-yl)biphenyl-4-carbaldehyde which comprises reacting 2'-cyanobiphenyl-4-carbaldehyde with a salt of azide.
<2> The process according to <1>, wherein the salt of azide is an azide of organic base.
<3> The process according to <2>, wherein the azide of organic base is the one prepared by an azide of inorganic base and a salt of organic base in the reaction system.
<4> The process according to any of <1> to <3>, wherein 2'-cyanobiphenyl-4-carbaldehyde is obtained by reacting 2'-cyano-4-(bromomethyl)biphenyl and/or 2'-cyano-4-(dibromomethyl)biphenyl with hexamethylenetetramine, acetic acid and water, and 2'-cyano-4-(bromomethyl)biphenyl and/or 2'-cyano-4-(dibromomethyl)biphenyl is obtained by brominating 2'-cyano-4-methylbiphenyl.
<5> The process according to <4>, wherein the bromination is conducted by bromine in the presence of a radical initiator and an oxidizer
<6> The process according to any of <1> to <5>, wherein the reaction of 2'-cyanobiphenyl-4-carbaldehyde with the salt of azide is conducted in a solvent.
<7> The process according to any of <1> to <6>, which further comprises obtaining a crystal of 2'-(1H-tetrazol-5-yl)biphenyl-4-carbaldehyde after the reaction.
<8> The process according to <7>, which further comprises, after the obtainment of the crystal, dissolving said crystal and recrystallizing a highly pure crystal in tetrahydrofuran.
<9> A process for producing a highly pure crystal of 2'-(1H-tetrazol-5-yl)biphenyl-4-carbaldehyde which comprises reacting 2'-cyanobiphenyl-4-carbaldehyde with a salt of azide, obtaining a crystal of 2'-(1H-tetrazol-5-yl)biphenyl-4-carbaldehyde, dissolving said crystal obtained and recrystallizing a highly pure crystal in tetrahydrofuran.
<10> A process for purifying a crystal of 2'-(1H-tetrazol-5-yl)biphenyl-4-carbaldehyde which comprises dissolving a crude crystal of 2'-(1H-tetrazol-5-yl)biphenyl-4-carbaldehyde containing 2'-(1H-tetrazol-5-yl)biphenyl-4-carboxylic acid, and recrystallizing a highly pure crystal of 2'-(1H-tetrazol-5-yl)biphenyl-4-carbaldehyde substantially containing no 2'-(1H-tetrazol-5-yl)biphenyl-4-carboxylic acid in tetrahydrofuran.
<11> A highly pure crystal of 2'-(1H-tetrazol-5-yl)biphenyl-4-carbaldehyde substantially containing no 2'-(1H-tetrazol-5-yl)biphenyl-4-carboxylic acid.
<12> A highly pure crystals of 2'-(1H-tetrazol-5-yl)biphenyl-4-carbaldehyde wherein 2θ of the crystal has main peaks at 9.2, 20.6, 25.7 and 26.9 when analyzed by X-ray diffraction analysis.

### BEST MODES FOR CARRYING OUT THE INVENTION

The present invention will be illustrated in detail below.

The present invention is a process for producing 2'-(1H-tetrazol-5-yl)biphenyl-4-carbaldehyde (hereinafter, may be referred to as TBAL), comprising a step of reacting 2'-cyanobiphenyl-4-carbaldehyde of the formula (I) (hereinafter, may be referred to as CBAL) with an salt of azide (hereinafter, may be referred to as Step (c)). If necessary, a high purity crystal of TBAL can be obtained by a step subjecting a TBAL crude crystal obtained by crystallization in Step (c) to dissolution into tetrahydrofuran and to recrystallization (hereinafter, referred to as Crystallization Step), in addition to said process above.

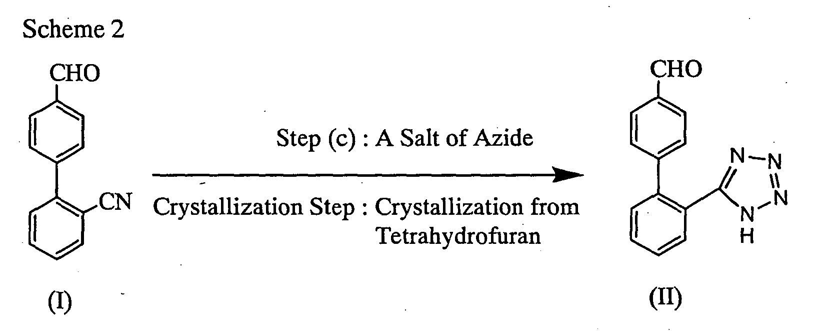

### 1. Step (c)

Step (c) is carried out, for example, by adding CBAL and an salt of azide in a solvent and stirring the mixture with heating.

Examples of the salt of azide used in Step (c) include azides of inorganic base and azides of organic base.

Examples of the azide of inorganic base include sodium azide, potassium azide, cesium azide and the like, and sodium azide is preferable because of its inexpensiveness.

Examples of the azide of organic base include triethylammonium azide, tri-n-propylammonium azide, tri-n-butylammonium azide and the like, and preferably mentioned are triethylammonium azide, tri-n-propylammonium azide and the like.

The amount of the salt of azide used is preferably 2.5 mol to 3.5 mol, more preferably 2.5 mol to 3.3 mol per 1 mol of CBAL from the standpoint of completion of the reaction and prevention of increase in the amount of a nitrite and the like added for excess salt of azide decomposition.

As the salt of azide, azide of organic bases are preferable from the standpoint of dissolvability in a solvent, and it is further preferable to prepare an azide of organic base from an azide of inorganic base and a salt of organic base in the reaction system since higher yield is obtained.

An azide of organic base can be prepared in the reaction system by adding an azide of inorganic base and a salt of organic base together with CBAL into a solvent.

When an azide of organic base is prepared, preferable azide of inorganic base is sodium azide and preferable salts of organic base are inorganic acid salts of trialkylamines such as triethylamine hydrochloride, tri-n-propylamine hydrochloride, tri-n-butylamine hydrochloride and the like.

The amount of an azide of inorganic base used in preparing an azide of organic base is the same as the above-mentioned salt of azide amount used, and the amount of a salt of organic base used is preferably 0.8 mol to 1.5 mol, more preferably 1 mol to 1.2 mol per 1 mol of an azide of inorganic base from the standpoint of securement of sufficient preparation of an azide of organic base and prevention of disturbance of stirring owing to increase of solid components in the reaction system.

The solvent used in Step (c) is not particularly limited as long as it does not disturb the reaction, and examples thereof include monochlorobenzene, methyl isobutyl ketone (MIBK), butyl acetate, diglime, dimethyl sulfoxide, N,N-dimethylformarnide and the like. These can be used singly or in combination of two or more. Preferably, monochlorobenzene and butyl acetate are mentioned.

The amount of a solvent used is usually 1000 to 2000 g, more preferably 1200 to 1500 g per 1 mol of CBAL.

The reaction temperature of Step (c) is usually in the range from 90 to 120°C, preferably from 100 to 115°C. The reaction time is usually 6 to 12 hours.

After completion of the reaction in Step (c), it is necessary to decompose an excess salt of azide. As the method of decomposing an salt of azide, a method of decomposition by nitrous acid is preferable.

When nitrous acid is used, it is preferable to prepare nitrous acid from a nitrite and an acid.

Specifically, nitrous acid can be prepared in the reaction system by, after completion of the reaction of Step (c), cooling the reaction mixture and adding a nitrite and an acid.

Examples of the nitrite include sodium nitride, potassium nitrite, calcium nitride and the like, and sodium nitrite is preferable from the economical standpoint.

Examples of the acid for generating nitrous acid include inorganic acids such as hydrochloric acid, sulfuric acid, phosphoric acid and the like, and hydrochloric acid is preferable.

In producing nitrous acid by an acid, it is preferable to control pH of the reaction system with an acid. In this case, it is preferable to control pH at from 3 to 7, and it is more preferable to control pH at 4.5±0.5 from the standpoint of prevention of generation of impurities or of safety.

In decomposing an salt of azide, it is desirable to add a hydrophilic solvent for progressing the decomposition reaction smoothly and suppressing generation of impurities. Examples of the hydrophilic solvent include tetrahydrofuran, acetone and the like, and tetrahydrofuran is preferable.

The amount of the hydrophilic solvent used is usually 1500 to 2500 g, preferably 1800 to 2200 g per 1 mol of CBAL. The decomposition temperature of an salt of azide is usually from 10 to 40°C.

TBAL obtained in Step (c) can be isolated by a conventional isolation method (concentration, extraction and the like), and it is preferable to isolate a TBAL crude crystal by the following method.

Here, "TBAL crude crystal" means a crystal isolated in Step (c), and when the crystal is analyzed by high performance liquid chromatography (HPLC), the area percentage is about 90% to 95%.

After completion of decomposition of an salt of azide, an aqueous layer is removed by phase-separation, and an organic layer is concentrated to an extent of not precipitation of a crystal to remove a hydrophilic solvent added.

Thereafter, a crystal is precipitated by cooling the mixture gradually (cooling rate: about 10°C/hour), and aged for about 2 to 40 hours at around 0 to 5°C. The precipitated crystal can be filtrated, washed and dried, to isolate a TBAL crude crystal.

### 2. Crystallization Step

Since the TBAL crude crystal obtained in Step (c) contains, as an impurity, an oxidized compound 2'-(1H-tetrazol-5-yl)biphenyl-4-carboxylic acid (hereinafter, referred to as TBCA) and the like, its purity is lower for a synthetic intermediate of medicines, and it is necessary to further purify the crystal for practical use.

When the TBAL crude crystal is dissolved in tetrahydrofuran before re-crystallization from this solution, impurities contained can be removed effectively, and particularly, a TBAL high purity crystal containing substantially no TBCA which is a main impurity can be obtained.

In the TBAL high purity crystal, "containing substantially no TBCA" means that when analyzed by HPLC, the area percentage of TBCA is 0.1% or less. "TBAL high purity crystal" means that TBAL has an area percentage of 99.5% or more.

Crystallization Step can be conducted by dissolving a TBAL crude crystal in tetrahydrofuran, then, gradually cooling the solution under stirring to precipitate a crystal, and further aging the crystal.

In Crystallization Step, the amount of tetrahydrofuran used is preferably 480 to 700 parts by weight, more preferably 490 to 650 parts by weight per 100 parts by weight of a TBAL crude crystal from the standpoint of prevention of mixing of impurities, recovery ratio, and the like.

The temperature at which a TBAL crude crystal is dissolved in tetrahydrofuran is preferably in the range from 55°C to 68°C.

After dissolving a TBAL crude crystal in tetrahydrofuran, crystallization may directly follow, alternatively, the heated solution before crystallization may be filtrated for removing contaminants.

In precipitating a crystal by cooling, it is preferable to add a seed crystal [TBAL] for stabilizing crystal growth and improving an effect of removing impurities.

The amount of the seed crystal to be added is usually 0.01 to 1 wt%, preferably 0.05 to 0.3 wt% based on the TBAL crude crystal.

The temperature at which the seed crystal is added is preferably 50°C to 55°C. When the seed crystal is added, a crystal is immediately precipitated, therefore, it is preferable to stir the solution for 2 to 6 hours at the temperature. At this stage, a crystal of about 70 to 95% of the total amount is precipitated.

For crystallization with good yield, further cooling to about 0 to 5°C is advantageous, and it is preferable to gradually cool the solution down to the temperature at a cooling rate of about 10°C/hour.

For further improving the yield, it is preferable to keep the solution at 0 to 5°C, and the keeping time is, for example, about 6 to 12 hours.

In filtrating the precipitated crystal, filtration is preferably conducted with cooling (specifically, at 0 to 5°C) for preventing re-dissolution of the crystal, and it is preferable to use a solvent cooled to the temperature as a washing solvent in washing after filtration.

Examples of the washing solvent include tetrahydrofuran and acetonitrile, and acetonitrile is preferable.

The amount of the washing solvent used is preferably 30 to 80 parts by weight, more preferably 40 to 60 parts by weight based on 100 parts by weight of a TBAL crude crystal.

The filtrated and washed crystal is dried at 50°C or less, preferably at 40 to 48°C under reduced pressure, to obtain "TBAL high purity crystal".

The TBAL crystal obtained in Crystallization Step is that of high purity containing substantially no TBCA and having a HPLC area percentage of 99.5% or more.

The TBAL high purity crystal shows a novel physical property.

For example, it shows an endothermic peak around 195°C in DSC analysis (analysis by differential scanning calorimeter), and in XRD analysis (powder X-ray diffraction method), diffraction angle 2θ(°) has a main peak at 9.2, 20.6, 25.7 and 26.9.

In the present invention, DSC value (value by differential scanning calorimetry) shows a value measured by Shimadzu DSC-60 (manufactured by Shimadzu Corp.) and XRD value (value by X-ray diffraction) shows a value measured by Rigaku Miniflex (manufactured by Rigaku Denki K.K.).
"shows an endothermic peak around 195°C in DSC analysis" means that when a crystal is analyzed by Shimadzu DSC-60, a single endothermic peak is shown at any temperature from 190 to 200°C.

As CBAL which is a raw material of Step (c), those produced by known methods (methods described in, for example, Synlett (2001), (12), 1893-1896, Organic Letters (2001), 3(10), 1435-1437, JP-H11-171802-A, Journal of the American Chemical Society (1995), 117(48), 11999-2000, Tetrahedron Letters (1994), 35(50), 9391-4, EP 606065 A1, US 53807, EP 443983 A1, Bioorganic & Medicinal Chemistry Letters (1993), 3(12), 2667-70, and the like) can be used, and it is preferable to produce CBAL by a method shown in the following reaction scheme.

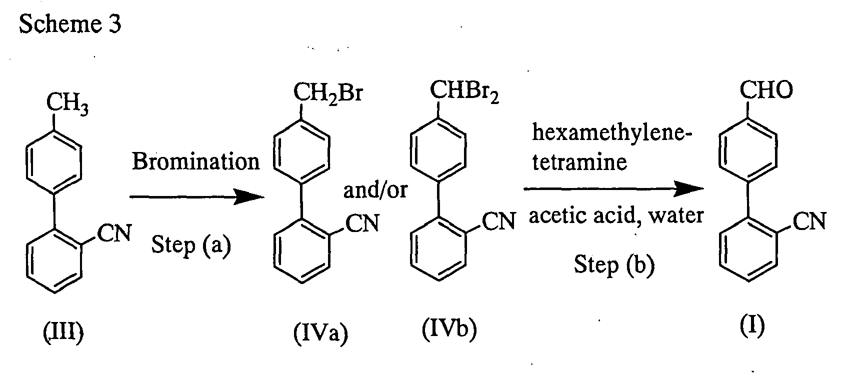

Namely, CBAL can be produced by a step of brominating 2'-cyano-4-methylbiphenyl (hereinafter, may be referred to as CMB) to produce 2'-cyano-4-(bromomethyl)biphenyl (hereinafter, may be referred to as CMBMB) and/or 2'-cyano-4-(dibromomethyl)biphenyl (hereinafter, may be referred to as CDBMB) (hereinafter, referred to as Step (a)) and a step of reacting the resultant CMBMB and/or CDBMB with hexamethylenetetramine, acetic acid and water (hereinafter, may be referred to as Step (b)).

In producing a monobromo compound CMBMB in bromination in Step (a), a dibromo body CDBMB is by-produced, while in Step (b), both CMBMB and CDBMB can be converted into CBAL by reacting with hexamethylenetetramine, and strict control of the bromination reaction is not necessary. Therefore, the process is advantageous.

### 3. Step (a)

In Step (a), bromination can be conducted by various methods. For example, it can be conducted by a combination with a brominating agent such as N-bromosuccinimide, bromine and the like in the presence of a radical initiator (see, JP-H08-127562-A), however, a method of reacting with bromine in the presence of a radical initiator and an oxidizer (hereinafter, referred to as Step (a-1)) suggested by the present inventors is preferable.

In Step (a-1), hydrogen bromide by-produced by bromination can be regenerated into bromine by co-existence of an oxidizer, as a result, disturbance of the reaction by hydrogen bromide can be prevented, and the amount of bromine used can be reduced. Therefor, the method is economically advantageous. Step (a-1) will be illustrated below.

Step (a-1) can be performed, for example, by reacting CMB with bromine in the presence of a radical initiator and an oxidizer in a solvent. The addition order of reagents is not particularly limited, and from the standpoint of operability, the reaction is preferably previously charged in a solvent, bromine and radical initiator or solutions thereof are added simultaneously. For smooth progress of the reaction, it is preferable to carry out the reaction under stirring.

In Step (a-1), the amount of bromine used is usually 0.4 mol or more, preferably 0.4 to 0.9 mol, further preferably 0.75 to 0.85 mol per 1 mol of CMB from the standpoint of prevention of yield decrease in the subsequent process by prevention of remaining of unreacted raw materials.

CMB which is a raw material of Step (a-1) can be produced by known methods, for example, methods described in J. Med. Chem. 1991, 34, 2525-2547, JP-H04-244080-A, JP-H04-253949-A and JP-H06-9536-A, and the like.

As the radical initiator, radical initiators such as azobis-based compounds, peroxides and the like are used. Specific examples of the azobis-based compound include 2,2'-azobis(4-methoxy-2,4-dimethylvaleronitrile), 2,2'-azobisisobutyronitrile, 2,2'-azobis(2-methylbutyronitrile), 2,2'-azobis(2,4-dimethylvaleronitrile); examples of the peroxide include dibenzoyl peroxide, di-t-butyl peroxide and the like. 2,2'-Azobis(2-methylbutyronitrile) and 2,2'-azobis(2,4-dimethylvaleronitrile) are preferred, and, 2,2'-azobis(2-methylbutyronitrile) is particularly preferred.

The amount of the radical initiator used is usually 0.1 to 10 mol%, preferably 1 to 4 mol% based on CMB as a raw material from the standpoint of reaction speed, and an effect compatible with addition amount, and the like.

The oxidizer includes oxidizers of relatively safe handling, for example, bromates such as sodium bromate, potassium bromate and the like; chlorates such as sodium chlorate, potassium chlorate and the like, and preferably sodium bromate is mentioned.

The amount of the oxidizer used may be advantageously a theoretically necessary amount for regenerating hydrogen bromide by-produced into bromine, or its slight excess amount, and is usually 10 to 30 mol%, preferably 17 to 25 mol% based on CMB as a raw bromine, an effect compatible with addition amount, and the like.

Examples of the solvent used in the Step (a-1) include halogenated hydrocarbons such as methylene chloride, ethylene dichloride, carbon tetrachloride, monochlorobenzene, o-dichlorobenzene, bromobenzene and the like; alkanes having 5 to 7 carbon atoms such as hexane, heptane, cyclohexane and the like; aliphatic esters such as methyl acetate, ethyl acetate, methyl propionate, ethyl propionate and the like; and monochlorobenzene are preferred.

The amount of the solvent used is usually 0.5 to 20 parts by weight, preferably 1 to 20 parts by weight, more preferably 1 to 15 parts by weight per 1 part by weight of CMB as a raw material from the standpoint of stirring efficiency, reaction speed and the like.

It is preferable that water is contained in the reaction system in Step (a-1). By containing water, the stirring efficiency increases steeply, and the reaction can be progressed smoothly. The water content is preferably 1 to 4 parts by weight, more preferably 1.5 to 2.5 parts by weight per 1 part by weight of the oxidizer.

The reaction temperature of Step (a-1) varies depending on the radical initiator and the like, and usually 50 to 100°C, preferably 50 to 85°C, more preferably 60 to 85°C. The radical initiator can also generate a radical by irradiation with light. In this case, a mercury lamp and the like can be used. The reaction time is also appropriately determined depending on the above-mentioned various reaction conditions (for example, about 3 to 10 hours).

CMBMB and/or CDBMB obtained in Step (a-1) can be isolated and purified from the reaction mixture by a conventional method. For example, after removal of an inorganic salt by filtration and the like, if necessary, a solvent is distilled off, and crystallization is performed using another suitable solvent, and the like. Also, a reaction mixture may be subjected to the subsequent reaction without particular isolation and purification.

### 4. Step (b)

In Step (b), CBAL can be produced, for example, by reacting CMBMB and/or CDBMB (hereinafter, both compounds may collectively be referred as Brominated Compounds) obtained in Step (a) with hexamethylenetetramine, acetic acid and water.

As Brominated Compounds which are a raw material, that which has been isolated and purified may be used, and the reaction solution in Step (a) may be used as it is.

A case using an isolated and purified Brominated Compounds (hereinafter, referred to as Step (b-1)) and a case using a reaction mixture in Step (a) (hereinafter, referred to as Step (b-2)) will be illustrated below.

### 4-1. Step (b-1)

As the solvent used in Step (b-1), ethanol and the like are mentioned in addition to the solvents used in Step (a). The amount of the solvent used is usually 2 to 3 parts by weight per 1 part by weight of Brominated Compounds.

In Step (b-1), water functions also as a solvent in addition to the reaction reagent, and is usually used in an amount of 500 to 600 g per 1 mol of a Brominated Compounds.

Also acetic acid functions as a solvent like water, and is usually used in an amount of 400 to 500 g per 1 mol of Brominated Compounds.

The amount of hexamethylenetetramine used is usually 1.5 to 3 mol, preferably 1.8 to 2.5 mol per 1 mol of Brominated Compounds.

The reaction temperature is usually 80 to 103°C, preferably 90 to 100°C.

A time when the area percentage of Brominated Compounds as a raw material in the reaction liquid reaches 0.5% or less in HPLC may advantageously be set as an end point, for example, and the reaction time is usually 8 to 14 hours.

### 4-2. Step (b-2)

As the solvent used in Step (b-2), ethanol and the like are mentioned in addition to the solvents used in Step (a). The total use amount of the solvents including the solvents used in Step (a) is usually 800 to 1000 g, preferably 850 to 900 g per 1 mol of CMB used in Step (a).

In Step (b-2), water functions also as a solvent in addition to the reaction reagent, and is usually used in an amount of 200 to 350 g, preferably 250 to 300 g per 1 mol of CMB used in Step (a).

Also acetic acid functions as a solvent like water, and is usually used in an amount of 350 to 500 g, preferably 400 to 450 g per 1 mol of CMB used in Step (a).

The amount of hexamethylenetetramine used is usually 2 to 4 mol, preferably 2.5 to 3.5 mol per 1 mol of CMB used in Step (a).

The reaction temperature is usually 80 to 103°C, preferably 90 to 100°C.

A time when the area percentage of Brominated Compounds as a raw material in the reaction liquid reaches 0.5% or less in HPLC may advantageously be set as an end point, for example, and the reaction time is usually 10 to 14 hours.

CBAL obtained in Step (b-1) or Step (b-2) can be isolated (e.g., concentration, extraction and the like) and purified (e.g., re-crystallization, silica gel chromatography, and the like) by ordinary methods, and also can be isolated and purified by treating according to the following method.

After completion of the reaction, the solution is allowed to stand still at 85 to 95°C to separate and remove an aqueous layer, a dilute alkaline aqueous solution (for example, potassium carbonate aqueous solution, sodium carbonate aqueous solution, sodium hydrogen carbonate aqueous solution, and the like) is used to control pH at from 7 to 8 and an organic layer is washed. Next, the solution is allowed to stand still, an aqueous layer is separated and removed, an organic layer is concentrated, and the residue is recrystallized from monochlorobenzene, toluene, ethyl acetate and the like. By the method, CBAL can be purified.

When monochlorobenzene is used as a reaction solvent, it is preferable to crystallize CBAL from monochlorobenzene by partially concentrating an organic layer.

In the case, concentration may advantageously be conducted so that the amount of monochlorobenzene becomes usually from 580 g to 630 g per 1 mol of a raw material Brominated Compounds or CBAL.

The TBAL high purity crystal produced by the present invention can be led to an angiotensin II antagonist of high quality, for example, by the method described in the above mentioned JP-H05-271205-A and the like.

The present invention will be illustrated further specifically by the following examples, but the present invention is not limited by them at all.

NMR (nuclear magnetic resonance spectrum) shows a value measured by JNM-AL400 (manufactured by JEOL Ltd.) and IR (infrared absorption spectrum) shows a value measured by PerkinElmer Spectrum 1000 (manufactured by PerkinElmer).

### Example 1: production of CBAL

To monochlorobenzene (1000 g)was added CMBMB (400 g, 1.47 mol), then, water (812 g), hexamethylenetetramine (412 g, 2.94 mol) and acetic acid (618 g, 10.29 mol) were added, then, the solution was stirred at 93°C for 9 hours. The solution was allowed to stand still for 1 hour at 85 to 90°C and phase-separation was conducted. To organic layer was added water (795 g), then, 10% potassium carbonate aqueous solution (684 g) was added to control pH at from 7 to 8. The solution was allowed to stand still for 1 hour, then, phase-separation was conducted. 142 ml of monochlorobenzene was distilled off at 85 to 95°C under a reduced pressure of 22.7 to 33.3 kPa. The solution was stirred at 70°C for 2 hours to cause growth of crystals, then, cooled down to 0 to 5°C at a rate of 10°C/hour and stirred at the same temperature for 5 hors. Filtration is performed, and the resultant crystals were washed with monochlorobenzene (400 g) cooled to about 5°C, and dried at about 60°C to obtain CBAL (235.6 g). The yield was 77.3%.

¹H-NMR (400 MHz, CDCl₃, dppm), 7.5 (2H, m, Ph), 7.6 (1H, m, Ph), 7.7 (2H, d, Ph), 7.8 (1H, m, Ph), 8.0 (2H, d, Ph),10.1 (1H, s, CHO)

IR (KBr) v 2224,1687 cm⁻¹

### Example 2: Production of CBAL

(1) To monochlorobenzene (450 g) was added CMB (300 g, 1.55 mol), and solution prepared by dissolving sodium bromate (50.1 g, 0.33 mol) in 95.3 g of water was added. 2,2'-azobis(2-methylbutyronitrile)(2.0 g, 0.01 mol) was dissolved in monochlorobenzene(10g), and this solution was added to the above-mentioned solution at 75 to 85°C, then, immediately, solution prepared by dissolving 2,2'-azobis(2-methylbutyronitrile)(89 g, 0.05 mol) in monochlorobenzene (48.8 g), and bromine (198.5 g, 1.24 mol) were dropped simultaneously respectively at 70 to 80°C. The solution of 2,2'-azobis(2-methylbutyronitrile) was dropped at a rate of about 0.22 g/min and bromine was dropped at a rate of about 0.73 g/min. The added solution was stirred for 5 hours at 70 to 75°C, and the reaction solution was checked under HPLC analysis conditions (1), and that the area percentage of raw materials reached 1% or less was confirmed, obtaining a mixed solution of CMBMB and CDBMB. The solution was analyzed under the HPLC analysis conditions (1), to find an area percentage of CMBMB of 63.6%, CDBMB of 36.2% and a raw material CMB of 0.2%.
(2) To monochlorobenzene (720 g) was added water (420 g), acetic acid (662 g) and hexamethylenetetramine (653 g, 4.66 mol), then, the whole amount of the brominated reaction solution of CMB obtained above was added, and the above-mentioned reaction vessel was washed with monochlorobenzene (120 g), and the washing solution was added to the reaction solution. The resulting solution was stirred at about 90°C for 11 hours. The stirred solution was allowed to stand still at 85 to 90°C for 1 hour, and aqueous layer was separated and removed. To organic layer was added water (840 ml), pH was adjusted to 7.8 with 10% potassium carbonate aqueous solution (540g), and the solution was allowed to stand still. Aqueous layer was separated and removed, then, 150 ml of monochlorobenzene was distilled off under a reduced pressure of 40 to 50 kPa at 85 to 90°C.

The resulting solution was cooled to 75°C, seed crystals were added, then, the mixture was stirred at the same temperature for 2 hours. The mixture was cooled at a rate of 10°C/hour, and aged at 8 to 12°C for 6 hours. Filtration was performed, and the resulting crystals were washed with monochlorobenzene (420 g) cooled to about 5°C, and dried at about 60°C to obtain CBAL (250.9 g). The yield was 78%.

### HPLC analysis conditions (1)

Column: SUMIPAX-ODS-A212, internal diameter 6 mm, length 15 cm (manufactured by Sumika Chemical Analysis Service Ltd.); mobile phase: liquid A: 0.1% acetic acid water, liquid B: methanol, A:B=4:6→0:10 (40 minutes, linear density gradient), flow rate: 1.0 ml/min, detection: λ=254 nm

### Example 3: Production of TBAL crude crystals

To monochlorobenzene (8510 g) was added CBAL (1294 g, 6.24 mol) triethylamine hydrochloride (2579 g, 18.73 mol), and under a nitrogen atmosphere, sodium azide (1218 g, 18.73 mol) was added and the mixture was heated at about 110°C and stirred. Under HPLC analysis conditions (2), the reaction solution was checked, and when the area percentage of a raw material CBAL reached 1.0% or less, the solution was cooled to about 10°C. Tetrahydrofuran (12.64 kg) and water (4.79 kg) were added, then, 15% sodium nitrite aqueous solution (5.745 kg, 12.49 mol) was added. pH was controlled at 5.0±0.1 by dropwise addition of 17.5% hydrochloric acid (7.03 kg).

The solution was allowed to stand still, then, aqueous layer was separated and removed, and organic layer was concentrated at 35 to 45°C under a reduced pressure of 40 to 45 kPa. When the amount of the distilled liquid reached 12.2 kg (about 10% w/w, as amount of remaining liquid), concentration was completed, the concentrated liquid was cooled down to 0 to 5°C at a rate of 10°C/hour and aged at the same temperature for 5 hours. After filtration, crystals were washed with monochlorobenzene (1294 g) cooled to 0 to 5°C, and dried at 50°C or lower under reduced pressure (about 8 kPa), to obtain a TBAL crude crystal (1250 g). The yield was 80.0%.

The resultant TBAL crude crystal was analyzed under the HPLC conditions (2), to find an area percentage of 96.1%, and the area percentage of an impurity TBCA was 0.73%.

### HPLC analysis conditions (2)

Column: SUMIPAX-ODS-A212, internal diameter 6 mm, length 15 cm (manufactured by Sumika Chemical Analysis Service Ltd.); mobile phase: liquid A: 0.1 % acetic acid water, liquid B: acetonitrile, A:B=7:3→4:6 (40 minutes, linear density gradient → A:B=4:6, kept for 10 minutes), flow rate: 1.0 ml/min, detection: λ=254 nm

### Example 4: Production of TBAL high purity crystals

To tetrahydrofuran (7736 g) were added TBAL crude crystals (area percentage of TBAL: 96.1%, area percentage of TBCA: 0.73%)(1250 g, 5.00 mol), and the solution was heated at about 65°C under a nitrogen atmosphere. Dissolution was confirmed, then, the solution was filtrated, and washed with tetrahydrofuran (57.6 g). Crystals of TBAL (1.4 g) were added at about 55°C, the mixture was maintained at 50 to 55°C for 3 hours under a nitrogen atmosphere, and cooled to 0 to 5°C at a rate of 10°C/hour. The mixture was aged at the same temperature for 6 hours and filtrated, and the resulting crystals were washed with acetonitrile (498 g) cooled to 0 to 5°C. The crystals were dried at a temperature of 48 to 50°C, to obtain TBAL high purity crystals (1000 g). The purification yield was 80%.

The resultant TBAL high purity crystal was analyzed under the HPLC conditions (2) to find an area percentage of 99.5%, and a peak of TBCAwas not detected.

### DSC analysis (differential scanning thermal analysis):

The high purity crystal was DSC-analyzed by Shimadzu DSC-60 (manufactured by Shimadzu Corp.), to find an endothermic peak of 195°C.

### XRD analysis (powder X-ray analysis):

The resulting TBAL high purity crystal was subjected to XRD analysis under the following conditions.
Apparatus: Rigaku Miniflex (manufactured by Rigaku Denki K.K.)
Filter: Kß filter
Wavelength: Ka₁
XG target: Cu
Slit: divergence slit

It was found that in the high purity crystal, its 2θ in XRD analysis had main peaks at 9.2, 20.6, 25.7 and 26.9.

### Example 5: Production of TBAL crude crystals

To monochlorobenzene (50 g) was added CBAL (10 g, 0.05 mol) and triethylamine hydrochloride (19.9 g, 0.14 mol), and sodium azide (9.4 g, 0.14 mol) was added under a nitrogen atmosphere and the mixture was stirred with heating at about 105°C. The reaction solution was checked under HPLC analysis conditions (3), and when the area percentage of a raw material reached 0.8%, the solution was cooled to 25°C. Tetrahydrofuran (98 g) and water (37 g) were added, then, 15% sodium nitrite aqueous solution (44.4 g, 0.1 mol) was added. pH was controlled to 4.0 by dropwise addition of 17.5% hydrochloric acid (54.3 g).

The solution was allowed to stand still, then, aqueous layer was separated and removed, and organic layer was concentrated at 40 to 45°C under a reduced pressure of 40 kPa or less. When the amount of the remaining liquid reached 46.3 g, concentration was completed, the concentrated liquid was cooled down to 0 to 5°C at a rate of 10°C/hour and aged at the same temperature for 25 hours. After filtration, crystals were washed with monochlorobenzene (10 g) cooled to 0 to 5°C, and dried at 50°C or lower under reduced pressure, to obtain TBAL crude crystals (11:8 g). The yield was 97.9%.

The resultant crude crystal was analyzed under the HPLC conditions (3), to find a purity of 92.6% in terms of area percentage, and the area percentage of an impurity TBCA was 0.87%.

### HPLC analysis conditions (3)

Column: SUMIPAX-ODS-A212, internal diameter 6 mm, length 15 cm (manufactured by Sumika Chemical Analysis Service Ltd.); mobile phase: liquid A: 0.014% trifluoroacetic acid water, liquid B: acetonitrile, A:B=7:3→1:9 (40 minutes, linear density gradient), flow rate: 1.0 ml/min, detection: λ=254 nm

### Example 6: Production of TBAL high purity crystals

To tetrahydrofuran (147 g) were added TBAL crude crystals (area percentage of TBAL: 92.6%, area percentage of TBCA: 0.87%)(30 g, 0.14 mol), and the solution was heated at about 60°C under a nitrogen atmosphere. Dissolution was confirmed, then, seed crystals of TBAL (0.03 g) were inoculated at 55°C, the mixture was maintained at 50 to 55°C for 3 hours under a nitrogen atmosphere, and cooled to 0°C at a rate of 10°C/hour. The mixture was aged at the same temperature for 10 hours and filtrated, and the resulting crystals were washed with acetonitrile (12 g) cooled to 0 to 5°C, and the crystals were dried at a temperature of 50°C or lower under reduced pressure, to obtain TBAL high purity crystals (18.0 g). The purification yield was 60.0%.

The resultant high purity crystal was analyzed under the HPLC conditions (3) to find an area percentage of 99.7%, and the area percentage of an impurity TBCA was 0.05%. DSC analysis (differential scanning thermal analysis):

The high purity crystal was DSC-analyzed by Shimadzu DSC-60 (manufactured by Shimadzu Corp.), to find an endothermic peak of 195°C.

### XRD analysis (powder X-ray analysis):

The resulting high purity crystal was subjected to XRD analysis under the same conditions as described above, to find that its 2θ had main peaks at 9.2, 20.6, 25.7 and 26.9.

### Example 7: Production of TBAL crude crystal

To butyl acetate (193 g) was added CBAL (30 g, 0.15 mol) and triethylamine hydrochloride (59.8 g, 0.43 mol), and sodium azide (28.2 g, 0.43 mol) was added under a nitrogen atmosphere and the mixture was stirred with heating at about 105°C. The reaction solution was checked under HPLC analysis conditions (3), and when the area percentage of a raw material reached 0.13%, the solution was cooled to 25°C. Tetrahydrofuran (293 g) and water (111 g) were added, then, 15% sodium nitrite aqueous solution (133.2 g, 0.29 mol) was added. pH was controlled to 4.3 by dropwise addition of 17.5% hydrochloric acid (162.8 g).

The solution was allowed to stand still, then, aqueous layer was separated and removed, and organic layer was concentrated at 40 to 45°C under a reduced pressure of 40 kPa or less. When the amount of the remaining liquid reached 176 g, concentration was completed, the concentrated liquid was cooled down to 0 to 5°C at a rate of 10°C/hour and aged at the same temperature for 25 hours. After filtration, crystals were washed with butyl acetate (27 g) cooled to 0 to 5°C, and dried at 50°C or lower under reduced pressure, to obtain titled crystals (31.9 g). The yield was 93.0%.

The resultant crude crystal was analyzed under the HPLC conditions (3), to find an area percentage of 95.6%, and the area percentage of an impurity TBCA was 0.63%.

### Example 8: Production of TBAL crude crystal

To methyl isobutyl ketone (58 g) was added CBAL (10 g, 0.05 mol) and triethylamine hydrochloride (19.9 g, 0.14 mol), and sodium azide (9.4 g, 0.14 mol) was added under a nitrogen atmosphere and the mixture was stirred with heating at about 105°C. The reaction solution was checked under HPLC analysis conditions (3), and when the area percentage of a raw material reached 2.2%, the solution was cooled to 24°C. Tetrahydrofuran (98 g) and water (37 g) were added, then, 15% sodium nitrite aqueous solution (44.4 g, 0.1 mol) was added. pH was controlled to 4.0 by dropwise addition of 17.5% hydrochloric acid (54.3 g).

The solution was allowed to stand still, then, aqueous layer was separated and removed, and organic layer was concentrated at 40 to 45°C under a reduced pressure of 40 kPa or less. When the amount of the remaining liquid reached 46.3 g, concentration was completed, the concentrated liquid was cooled down to 0 to 5°C at a rate of 10°C/hour and aged at the same temperature for 21 hours. After filtration, crystals were washed with methyl isobutyl ketone (8 g) cooled to 0 to 5°C, and dried at 50°C or lower under reduced pressure, to obtain TBAL crude crystals (7.77 g). The yield was 64.3%.

The resultant crude crystal was analyzed under the HPLC analysis conditions (3), to find an area percentage of 92.3%, and the area percentage of an impurity TBCAwas 0.59%.

The present process is industrially advantageous since a crystal of 2'-(1H-tetrazol-5-yl)biphenyl-4-carbaldehyde which is useful as a synthetic intermediate of medicines can be produced in a short process at high yield and high purity.

## Claims

1. A process for producing 2'-(1H-tetrazol-5-yl)biphenyl-4-carbaldehyde which comprises reacting 2'-cyanobiphenyl-4-carbaldehyde with a salt of azide.

2. The process according to Claim 1, wherein the salt of azide is an azide of organic base.

3. The process according to Claim 2, wherein the azide of organic base is the one prepared by an azide of inorganic base and a salt of organic base in the reaction system.

4. The process according to Claim 1, wherein 2'-cyanobiphenyl-4-carbaldehyde is obtained by reacting 2'-cyano-4-(bromomethyl)biphenyl and/or 2'-cyano-4-(dibromomethyl)biphenyl with hexamethylenetetramine, acetic acid and water, and 2'-cyano-4-(bromomethyl)biphenyl and/or 2'-cyano-4-(dibromomethyl)biphenyl is obtained by brominating 2'-cyano-4-methylbiphenyl.

5. The process according to Claim 4, wherein the bromination is conducted by bromine in the presence of a radical initiator and an oxidizer.

6. The process according to Claim 1, wherein the reaction of 2'-cyanobiphenyl-4-carbaldehyde with the salt of azide is conducted in a solvent.

7. The process according to Claim 1, which further comprises obtaining a crystal of 2'-(1H-tetrazol-5-yl)biphenyl-4-carbaldehyde after the reaction.

8. The process according to Claim 7, which further comprises, after the obtainment of the crystal, dissolving said crystal and recrystallizing a highly pure crystal in tetrahydrofuran.

9. A process for producing a highly pure crystal of 2'-(1H-tetrazol-5-yl)biphenyl-4-carbaldehyde which comprises reacting 2'-cyanobiphenyl-4-carbaldehyde with a salt of azide, obtaining a crystal of 2'-(1H-tetrazol-5-yl)biphenyl-4-carbaldehyde, dissolving said crystal obtained and recrystallizing a highly pure crystal in tetrahydrofuran.

10. A process for purifying a crystal of 2'-(1H-tetrazol-5-yl)biphenyl-4-carbaldehyde which comprises dissolving a crude crystal of 2'-(1H-tetrazol-5-yl)biphenyl-4-carbaldehyde containing 2'-(1H-tetrazol-5-yl)biphenyl-4-carboxylic acid, and recrystallizing a highly pure crystal of 2'-(1H-tetrazol-5-yl)biphenyl-4-carbaldehyde substantially containing no 2'-(1H-tetrazol-5-yl)biphenyl-4-carboxylic acid in tetrahydrofuran.

11. A highly pure crystal of 2'-(1H-tetrazol-5-yl)biphenyl-4-carbaldehyde substantially containing no 2'-(1H-tetrazol-5-yl)biphenyl-4-carboxylic acid.

12. A highly pure crystal of 2'-(1H-tetrazol-5-yl)biphenyl-4-carbaldehyde wherein 2θ of the crystal has main peaks at 9.2, 20.6, 25.7 and 26.9 when analyzed by X-ray diffraction analysis.
